# NEW EUROPEAN PATENT SPECIFICATION

(11) **EP 0 134 048 B2**
(45) Date of publication and mention of the opposition decision: **14.08.1996**
(45) Mention of the grant of the patent: 08.11.1989
(21) Application number: 84200990.4
(22) Date of filing: 06.07.1984
(51) Int. Cl.: C12N 15/00, C12N 9/28, C12N 9/56, C12N 9/64

(54) **Molecular cloning and expression in industrial microorganism species**
Molekulare Klonierung und Expression in industriellen Mikroorganismen
Clonage moléculaire et expression dans des espèces de micro-organismes industriels

(30) Priority: 06.07.1983 EP 83201016
(43) Date of publication of application: 13.03.1985
(73) Proprietor: GIST-BROCADES N.V., NL-2600 MA Delft (NL)
(72) Inventor: Sanders, Johan Pieter Marinus, NL-2613 BV Delft (NL); van den Berg, Johannes Abel, NL-2811 AD Reeuwijk (NL); Andreoli, Peter Michael, NL-3055 EJ Rotterdam (NL); Vos, Yvonne Johanna, NL-1106 DL Amsterdam Z.O. (NL); van Ee, Jan Hendrik, NL-3068 BX Rotterdam (NL); Mulleners, Leo J.S.M., NL-5044 EH Tilburg (NL)
(74) Representative: Huygens, Arthur Victor, Dr.

(56) References cited:
- EP-A- 0 032 238
- EP-A- 0 034 470
- EP-A- 0 036 259
- EP-A- 0 060 663
- EP-A- 0 077 109
- GB-A- 2 091 268
- BIOLOGICAL ABSTRACTS, vol. 73, no. 8, 1982, no. 53368, Biosciences Information Service, Philadelphia, USA; X. JIANG et al.: "The transfer of plasmids through cell fusion in Bacillus subtilis" & ACTA GENET SIN 8(1), 1-7, 1981
- BIOLOGICAL ABSTRACTS, vol. 73, no. 6, 1982, no. 38805, Biosciences Information Service, Philadelphia, USA; L. MINGFENG et al.: "A study on plasmid pUB110 transfer by Bacillus subtilis protoplast fusion" & ACTA GENET SIN 8(2), 109-115, 1981
- CHEMICAL ABSTRACTS, vol. 96, no. 25, 21st June 1982, page 401, no. 214136e, Columbus, Ohio, USA; J.C. GROSCH et al.: "Transformation of Bacillus licheniformis and Bacillus amyloliquefaciens protoplasts by plasmid DNA" & GENET. CELL. TECHNOL. 1982, 1(GENET. EXCH.), 97-105
- AGRIC. BIOL. CHEM., vol. 47, no. 1, 1983, pages 159-161; Y. TAKEICHI et al.: "Cloning of Bacillus subtilis alpha-amylase structural gene in plasmid pUB110"
- JOURNAL OF BACTERIOLOGY, vol. 154, no. 2, May 1983, pages 831-837, American Society for Microbiology, USA; M. FUJII et al.: "Molecular cloning of a thermostable neutral protease gene from Bacillus stearothermophilus in a vector plasmid and its expression in Bacillus stearothermophilus and Bacillus s
- ACTA GENETICA SINICA (1981), vol. 8, pp. 1-7 and 109-115

## Description

### Background of the invention

### Field of the invention

There is substantial interest in employing industrial unicellular microorganism strains as hosts with recombinant DNA to produce polypeptides in high yields. Many industrially important enzymes, such as amylolytic and proteolytic enzymes, are produced by microorganisms of the genus *Bacillus,* e.g., *B*. *subtilis, B. amyloliquefaciens, B. licheniformis, B. steaerothermophilus* and *B. coagulans.* In fermenters, strains are employed which are highly robust and stable. Furthermore, the strains are resistant to phage infection and, in addition, to genetic exchange, that is introduction of DNA by conventional transformation procedures. The conventional industrial strains are also prototrophic, in order to avoid adding expensive amino acids to the nutrient medium. Other characteristics of industrial strains are their high productivity until the end of the fermentation, which can be as long as a week, stable cell concentration upon exhaustion of the broth, and high productivity, usually at least about 0.5% w/v secreted protein. In addition, it is often found with bacilli, that there is a substantial secretion of DNAses, so that there is substantial degradation of any DNA in the medium.

Due to the genetic modification resistant nature of the industrial strains and their prototrophic character which makes them difficult to starve, they show resistance to transformation. It would therefore be of great value to provide for an efficient process for introducing DNA into industrial strains, where the DNA would be stably maintained in the industrial strain, there would be no loss or substantially no loss of viability and activity of the industrial strain and high yields of endogenous and exogenous polypeptide or protein products could be obtained.

Furthermore, selection of cells is difficult where the modification or transformation of the host cells involves increasing the copy number of an endogenous gene or previously introduced gene, where the gene is not involved with survival selection. It is therefore highly desirable to have an efficient process in which the presence of additional genes (increased copy number) can be detected and selected for.

### Description of the prior art

Genetic manipulations of *B. subtilis* have been reported by Yoneda *et al., Biochem. Biophys. Res. Commun*. (1973) *50*:765-770; Yoneda and Maruo, *J. Bacteriol*. (1975) *124*: 48-54; Sekiguchi *et al*., *J*. *Bacteriol,* (1975) *121*:688-694; Hitotsuyanagi *et al., Agric. Biol. Chem.* (1979) *43*:2342-2349; Yoneda, *Appl. Env. Microbiol.* (1980) *39*:274-276. Successful applications of recombinant DNA technology with respect to production improvements of certain, efficiently transformable laboratory strains of *B. subtilis* have been reported, e.g. those producing alpha-amylases, beta-lactamases, dihydrofolate reductase, interferon and insulin (Palva, *Gene* (1982) *19*:81-87; Shinomiya *et al., Agric. Biol. Chem.* (1981) *45*:1733-1735; Gray and Chang, *J*. *Bacteriol.* (1981) *145*:422-428; Williams *et al., Gene* (1981) *16*:199-206; Palva, *Gene* (1983) *22*:229-235). The difficulties in genetically manipulating *Bacillus licheniformis* soil isolates is reported by Thorne and Stull, *J. Bacteriol.* (1966) *91*:1012-1014. See also, U.K. Patent Application GB2091268; European Patent Application 0 034 470; European Patent Application 0 032 238; and European Patent Application 0 077 109, which disclosure is incorporated herein by reference, as it relates to pUR1523.

Minfeng *et al* in Acta Genetica Sinica, Vol 8 (1981) 109-115 disclose the use of polyethylene glycol to induce protoplast fusion of aerobic bacilli as a means of transferring plasmid DNA into non-competent strains of bacilli. The method is used only with laboratory strains of bacilli not industrial strains. Similarly, Xingjuan *et al* in Acta Genetica Sinica Vol. 8 (1981) 1-7 have applied protoplast fusion of laboratory strains of B. subtilis in the presence of polyethylene glycol in order to obtain the transfer of plasmid DNA.

### Summary of the invention

Novel methods and products involving genetically modified industrial *Bacillus* strains, are provided. Extrachromosomal DNA containing a gene of interest capable of expression in an industrial strain host is introduced into an appropriate bacterial host, conveniently a laboratory strain host related to the industrial strain, and the modified bacterial host combined with an industrial strain under fusing conditions. Cells of the industrial strain containing the gene(s) of interest are selected by means of a marker associated with the gene of interest.

### Brief description of the drawings

Figure 1 is a diagrammatic view of the plasmid pGB33;
Figure 2 is a diagrammatic view of the plasmid pGB34;
Figure 3 is a diagrammatic view of the plasmid pLC28;
Figure 4 is a diagrammatic view of the plasmid pLC83;
Figure 5 is a diagrammatic view of the plasmid pLC87;
Figure 6 is a diagrammatic view of the plasmid pGB35;
Figure 7 is a diagrammatic view of the plasmid pLP33;
Figure 8 is a diagrammatic view of the plasmid pLP87; and
Figure 9 is a SDS polyacrylamide gel-electrophoretogram of products secreted by various *Bacillus* industrial strains relative to protein molecular weight markers.

### Description of the specific embodiments

Methods are provided for the genetic manipulation of industrial Bacillus strains for use in fermentation for the production of polypeptide products in good yield. The resulting modified industrial strains retain the desirable characteristics of industrial strains, while providing enhanced yields of expression products of endogenous (same species) or exogenous (different species) genes.

The method involves introducing extrachromosomal DNA into a bacterial host, which is capable of replicating the DNA and is readily susceptible to the introduction of the extrachromosomal DNA. The modified bacterial host cell containing the extrachromosomal element is then combined with the industrial *Bacillus* strain under fusing conditions, where the recipient *Bacillus* cells may be subsequently selected for the presence of the gene or genes of interest originating from the extrachromosomal element.

The subject invention may be divided up into the following parts: (1) preparation of the plasmid construct, including the gene(s) for which enhanced expression in the *Bacillus* host is desired; (2) cloning of the plasmid construct in a compatible host, which can be used for fusion with the industrial *Bacillus* strain; (3) fusion of the plasmid construct-containing host with the industrial *Bacillus* strain, including selection of such industrial strain; and (4) growing of said strain in an appropriate nutrient medium for production of the expression product of the gene(s) of interest.

The gene(s) of interest may be any prokaryotic or eukaryotic gene. These genes may include bacterial genes, unicellular microorganism genes, mammalian genes, or the like. The structural genes may be prepared in a variety of ways, including synthesis, isolation from genomic DNA, preparation from cDNA, or combinations thereof. The various techniques of manipulation of the genes are well-known, and include restriction, digestion, resection, ligation, *in vitro* mutagenesis, primer repair, employing linkers and adapters, and the like. Thus, DNA sequences obtained from a host may be manipulated in a variety of ways, depending upon the requirements of the DNA construction. See Maniatis *et al., Molecular Cloning,* Cold Spring Harbor Laboratory, Cold Spring Harbor, N.Y., 1982.

Where the gene is obtained from a host which has transcriptional and translational initiation and termination regulatory signals which are recognized by the industrial *Bacillus* strain, it will usually be convenient to maintain the 5'- and 3'-flanking regions of the structural gene to provide a cistron capable of expression in the industrial *Bacillus* host. The transcriptional initiation region may provide for constitutive or inducible expression, so that in appropriate situations, the host may be grown to high density before high levels of expression of the structural genes of interest are obtained.

Where the structural gene is from a source whose regulatory signals are not recognized by *Bacillus,* it will be necessary to obtain regulatory regions recognized by *Bacillus* and insert the structural genes between the initiation and termination regulatory signals. In some instances the exogenous structural gene with its own stop codon(s) may be inserted in reading frame behind the N-terminus codons of an endogenous *Bacillus* structural gene having its natural regulatory signals. The resulting product may then have some, for example, 0 to 30, additional N-terminal amino acids. Alternatively, operons can be prepared, where a single promoter provides for transcriptional initiation of a messenger RNA which codes for a plurality of polypeptides.

In some instances, it may be desirable that the expression product be secreted. Where the expression product is naturally secreted and the leader signal and processing signal(s) are recognized by the *Bacillus* host, this will entail no difficulty. However, where the product is not normally secreted, or *Bacillus* does not recognize the secretory signals and/or processing signal(s), that is the signals are not functional to a satisfactory degree in *Bacillus,* then it may be necessary to isolate or synthesize DNA sequences coding for the secretory signals and processing signal(s) of a *Bacillus* polypeptide and join them to the 5'- end of the structural gene in proper reading frame.

The structural genes may express a variety of polypeptides or proteins, such as enzymes, hormones, lymphokines, surface proteins, blood proteins, structural proteins, immunoglobulins, or the like, from mammals, unicellular microorganisms, e.g., bacteria, fungi, such as yeast, or filamentous fungi, algae, protozoa, etc., plants, or other DNA source. Of particular interest are enzymes, more particularly hydrolases and more particularly proteases and saccharidases. Illustrative of such enzymes are endopeptidases, exopeptidases, serine and non-serine proteases, alpha- and beta-amylases (particularly thermostable alpha-amylase), and the like.

There are a wide number of vectors which can be employed for the compatible host as well as the *Bacillus* strain, where the replication systems may be the same or different for the compatible host and the *Bacillus* strain. (By vector is intended a replication system(s) compatible with one or more hosts, usually a marker for selection in the host and at least one convenient, usually unique, restriction site). Usually, it will be convenient to have as the compatible host a non-industrial or laboratory *Bacillus* strain, although this is not necessary and in some instances other organisms may be used, such as *E. coli.* The vector will include one or more replication systems so that it is at least capable of being cloned in the compatible host. The replication system can provide for either high or low copy number, preferably a copy number of at least about 10 and generally not more than about 100.

Depending upon whether one desires integration of the structural genes in the industrial *Bacillus* strain or maintenance on an extrachromosomal element, a replication system for *Bacillus* may or may not be included. Alternatively, for integration, one may provide for stretches of homology in the vector or plasmid construct with the *Bacillus* genome to enhance the probability of recombination.

In addition to the replication system, there will be at least one marker and there may be more than one marker, usually not more than about three markers. By marker is intended a structural gene capable of expression in a host, which provides for survival selection. By "survival selection" is intended imparting prototrophy to an auxothrophic host, biocide or viral resistance. For prototrophy, various genes may be employed, such as leu, ura, trp, or the like. For biocide resistance this may include resistance to antibiotics, e.g. neo, cam, tet, tun, kan, or the like. Other markers include resistance to heavy metals, immunity, and the like. The various DNA sequences may be derived from diverse sources and joined together to provide for a vector which includes one or more convenient, preferably unique. restriction sites to allow for insertion or substitution of the structural genes at such sites or in place of lost fragments to provide the plasmid construct.

Once the plasmid construct has been prepared, it may now be cloned in an appropriate compatible host referred to as the compatible or cloning host. Any host may be used which is convenient, is readily transformable, and allows for replication of the plasmid construct and transfer to the industrial *Bacillus* strain through fusion. A large number of laboratory strains are available which have a high efficiency of transformation and are usually auxotrophic and/or antibiotic sensitive. The use of a *Bacillus* host for cloning of the plasmid construct has many advantages in that it permits the use of a single replication system as well as the same marker for survival selection in both the compatible host and the industrial strain. Thus, for the most part, the plasmid construct will be cloned in an appropriate *Bacillus* host. The *Bacillus* host need not be the same *Bacillus* strain as the industrial host and will be chosen primarily for convenience. The plasmid construct may be introduced into the compatible host in accordance with conventional techniques, such as transformation, employing calcium precipitated DNA, conjugation, or other convenient technique. The compatible host may then be grown in an appropriate nutrient medium, under selective conditions to select for a host containing the plasmid construct. For auxotrophic hosts, the nutrient medium is deficient in the required nutrient, while for biocide resistance, a cytotoxic amount of the biocide(s), e.g. antibiotic(s), is employed in the nutrient medium. After growing the compatible host to a sufficient density, the compatible host is then treated to prepare the cells for fusion.

The cells are killed with a cytotoxic agent prior to or during protoplast formation. Various agents may be employed, including antibiotics, but iodoacetamide is found to be convenient, efficient, and does not interfere with the subsequent fusion. Protoplasts are prepared from the cells in accordance with conventional ways, e.g., lysozyme or zymolyase treatment, and the protoplasts are carefully suspended in an appropriate medium having proper osmolality for maintaining the integrity of the protoplast.

The industrial *Bacillus* acceptor strain is also treated to form protoplasts in a similar manner as the compatible host strain, but viable cells are employed for preparing protoplasts. Various *Bacillus* strains may be employed which have the desired traits of an industrial *Bacillus* strain, such as *subtilis, licheniformis, amyloliquefaciens, stearothermophilus* and *coagulans,* preferably *licheniformis* and *subtilis.* The industrial *Bacillus* strains are chosen from organisms which may be isolated from the soil or available from depositories or other sources or obtained by modification of such *Bacillus* strains. The industrial *Bacillus* strains are characterized by being resistant to genetic exchange, such as phage infection or transformation. The strains are stable and may or may not be capable of spore formation. They are prototrophic and provide for high yields of endogenous protein products, such as the enzymes alpha-amylase and various proteases. They also are found to secrete DNAses, which results in the degradation of DNA in the medium, providing for protection against genetic exchange.

The dead compatible host protoplast and the viable industrial *Bacillus* host protoplast are combined in the presence of an appropriate fusogen. While any fusogen may be employed which provides a desired efficiency, for the most part polyethylene glycol Is found to provide high efficiency of fusion with great convenience. The ratio of the dead protoplast to the *Bacillus* acceptor strain will be preferably at least 1:1 and excesses of the dead protoplast may be employed. After a short time, the fusogen mixture is replaced with an appropriate nutrient medium and cells regenerated in a selective medium, conveniently by plating on an agar plate. The clones may then be screened in appropriate ways for detection of the expression of the additional structural genes. Various techniques may be employed, particularly where enzymes are involved which have well established methods of detection. Alternatively, where enzymes are not involved or there is no available detection system, antibodies, DNA or RNA hybridization, or bioassays can be employed for screening the clones to determine the presence of the plasmid construct and expression of the structural gene(s) of interest.

The industrial *Bacillus* host containing the plasmid construct or chromosomally integrated plasmid constructs or fragments thereof is then grown in a nutrient medium under conventional fermenting conditions. The fermenting may be continued until the broth is exhausted. Where the product has been secreted, the product may be isolated from the broth by conventional techniques, e.g., extraction, chromatography, electrophoresis, or the like. Where the product is retained in the cytoplasm, the cells maY be harvested by centrifugation, filtration, etc., lysed by mechanical shearing, detergent, lysozyme, or other techniques and the product isolated as described previously. By employing the subject method greatly enhanced yields of endogenous polypeptides can be achieved, usually at least about 150% the yield of the parent cell, more usually at least 175%, and preferably at least about 200% the yield of the parent cell.

The following examples are offered by way of illustration and not by way of limitation.

### Example I

### Isolation of chromosomal DNA

Chromosomal DNA of *B. licheniformis* industrial strain T5, deposited with Centraal Bureau vorr Schimmel-cultures, Oosterstraat 1, Baarn, the Netherlands (hereinafter CBS) on July 6, 1983 under No. 470.83, was isolated from 3L cultures, that were grown overnight at 37°C under aeration. Cells were spun down at 10,000 rpm for 10 min in a Sorvall GSA rotor, suspended in 10 ml sucrose-Tris buffer containing 25% w/v sucrose and 50 mM Tris-HCI pH 8.0, and lysed by addition of 0.5 ml lysozyme solution (20 mg/ml) and incubation for 15 min at 37°C. After addition of 2 ml EDTA (0.5 M) and incubation for 5 min at 0°C, 1 ml 20% (w/v) sodium dodecyl sulphate (SDS) was added. The suspension was then extracted with a 1:1 phenolchloroform mixture. The supernatant was separated and carefully overlayed by 2 volumes of pure ethanol after which the DNA was isolated with the aid of a glass rod. After dissolving in distilled water containing 10 µg/ml ribonuclease, the DNA suspension was extracted with 1:1 phenol-chloroform, precipitated with 2 volumes of ethanol and resuspended in TE buffer (i.e., 10 mM Tris-HCI, pH 8.0 containing 1 mM EDTA).

### Example II

### Isolation of plasmid DNA

*B. subtilis* strain 1G 20, containing plasmid pUB110 (cf. European Patent Specification 0 021 468), was grown overnight in 1L penassay broth medium to which 5 µl/ml neomycin had been added. After centrifuging for 15 min at 5000 rpm in a Sorvall model GSA rotor and resuspending in 15 ml sucrose-Tris buffer, the cells were lysed and treated with EDTA and SDS as described in Example I. After addition of NaCI to a final concentration of 1M the supernatant was stored overnight at 4°C and then centrifuged for 45 min at 12500 rpm in a Sorvall type SS 34 rotor. The upper 70% (v/v) of the supernatant was treated with 20 µg/ml DNAse-free RNAse (for 0.5h at 37°C), and extracted with phenolchloroform (1:1) mixture, followed by extraction with chloroform alone.

The DNA was precipitated from the extracted supernatant by addition of 0.2 volume of 5M NaCI and 0.25 volume of 40% (w/v) polyethylene glycol 6000, followed by incubation for 16 h at 4°C. After precipitation and centrifugation (30 min at 12500 rpm, Sorvall type SS 34 rotor) the DNA was resuspended in 2-3 ml TE buffer (as in Example I) and the dispersion made pH 12.0 with 4 M NaOH. The pH was then adjusted to 8.5 and the suspension was extracted with phenol. After precipitation of the extract with ethanol and plasmid DNA was resuspended in a small volume of TE buffer.

Plasmid pUR1523 (cf. European specification A-77109) DNA from *E. coli* was isolated according to the method described by Birnboim and Doly, *Nucl. Acids Res.* (1979) *7*: 1513-1523.

### Example III

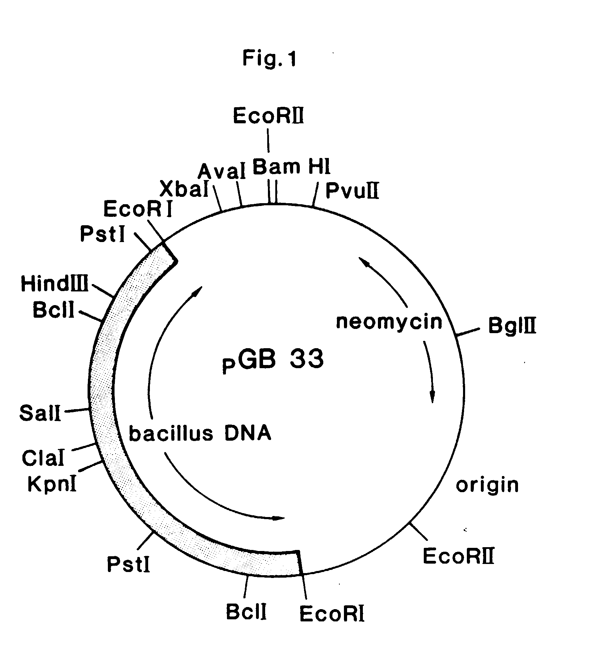

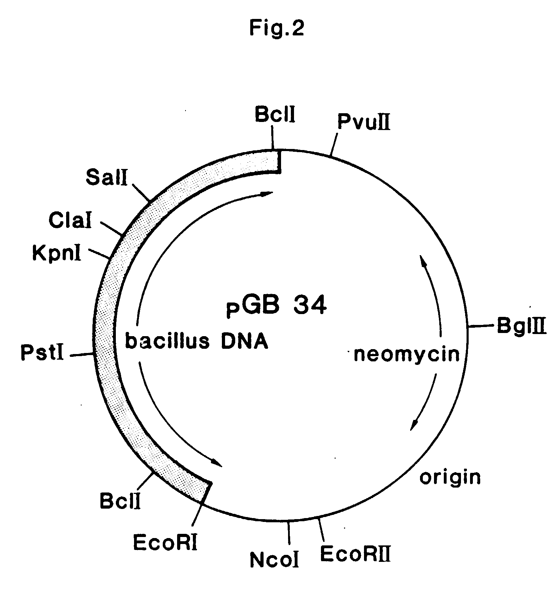

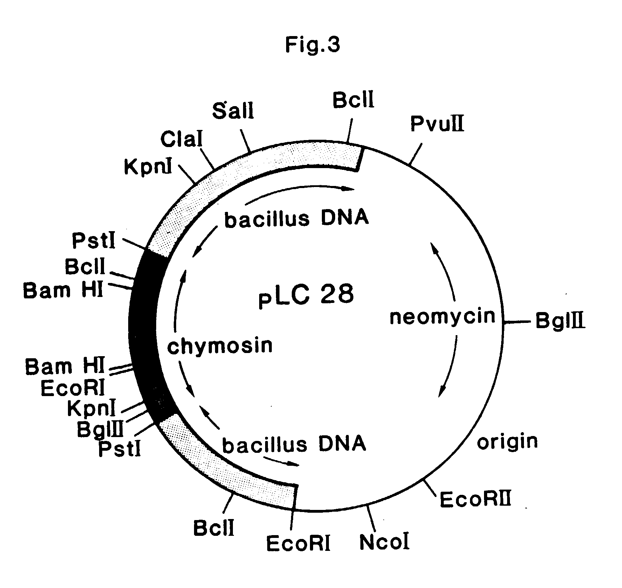

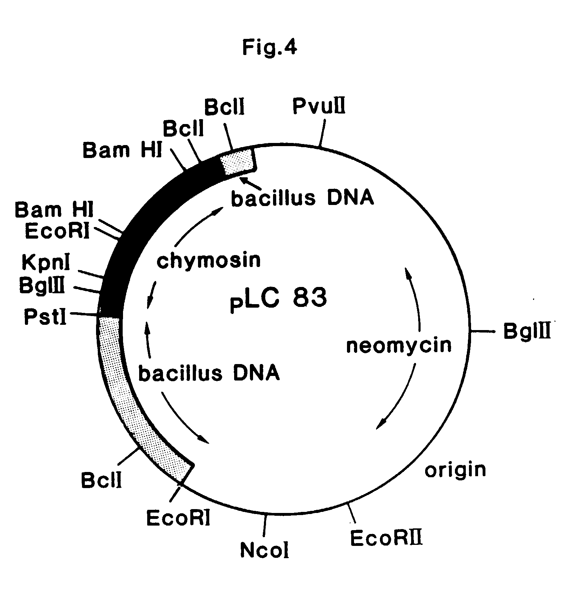

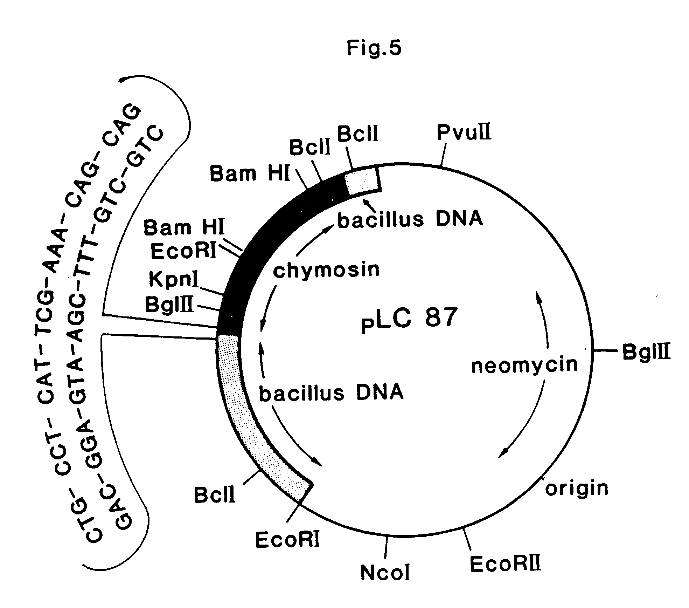

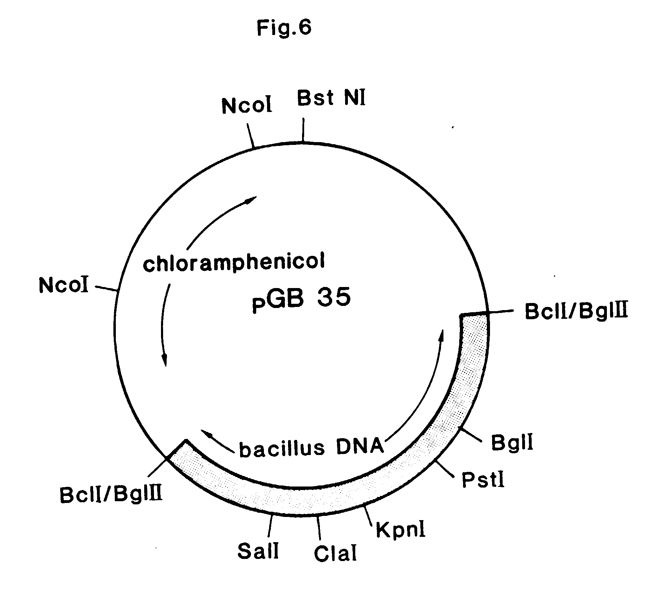

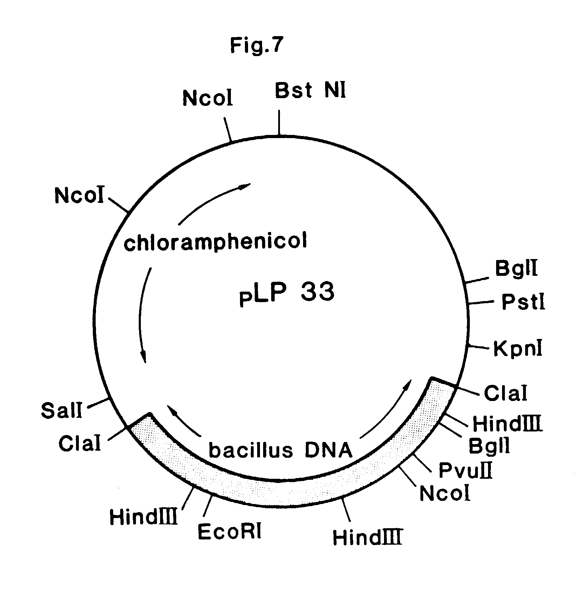

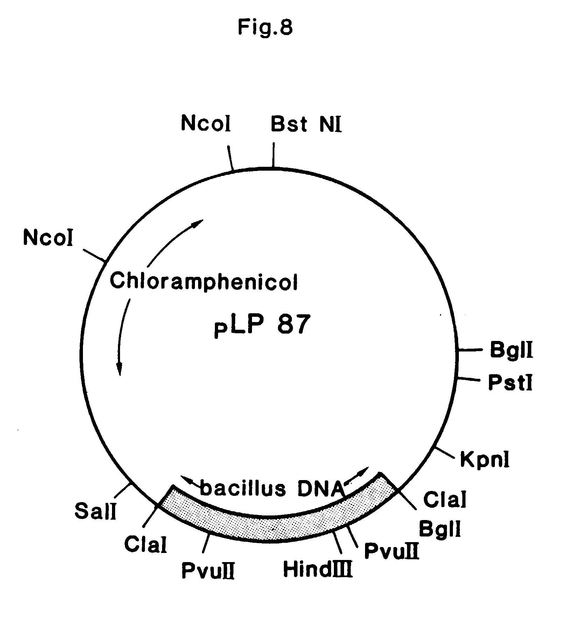

a) Construction of the recombinant plasmids pGB33 and pGB34 containing a gene encoding alpha-amylase (Figs. 1 and 2)
   5 µg chromosomal DNA, isolated from the *Bacillus* production strain T5 (as described in Example I) and 1 µg pUB 110, isolated from *B. subtilis* 1G 20 (as described in Example II) were digested with *Eco*RI. After thermal denaturation of the restriction enzyme for 7.5 min at 65°C, the DNA was precipitated with ethanol and resuspended in 20 µl of a ligase mixture containing 20 mM Tris-HCI pH 7.6, 10 mM MgCl₂, 10 mM dithiothreitol (DTT), 0.2 mg/ml bovine serum albumin, 0.5 mM ATP and 1 unit of T₄ ligase (Boehringer-Mannheim). The mixture was ligated overnight at 4°C. The ligated mixture was transferred into *B. subtilis* as described in Example IV below. Plasmid DNA was isolated (using the method described in Example II) from selected recombinant microorganisms and analysed with restriction endonucleases. Plasmid pGB33 was a recombinant of pUB110 and a chromosomal *Eco*RI fragment of approx. 3 kbp, containing the alpha-amylase cistron. Digesting pGB33 with the restriction endonucleases *Hind*III and *Bam*HI, followed by S₁ exonuclease resection and ligation with T₄ yielded pGB34 (Fig. 2), which still harbors the alpha-amylase cistron but lacks a DNA segment containing many inconvenient restriction sites. The plasmids pGB33 in *B. subtilis* 1-85 (=trp⁻) and pGB34 in *B. subtilis* 1S-53 (Bacillus Genetic Stock Center, Ohio, U.S.A.) were deposited with CBS on July 6, 1983 as Nos. 466.83 and 467.83, respectively.
b) Construction of the recombinant plasmids pLC28, pLC83 and pLC87 containing a gene encoding chymosin (Figs. 3, 4 and 5)
   0.5 µg pGB34 DNA and 0.5 µg pUR1523, an *E. coli* plasmid that harbors the gene for bovine chymosin, were digested with *Pst*I. After thermal denaturation and ligation as described in Example IIIa, the mixture was used for transformation. Plasmid DNA isolated from selected transformants (using the selection procedure described in Example IV below) was analyzed with restriction endonucleases. The selected plasmid, called pLC28, was a recombinant of the chymosin gene containing *Pst*I fragment of pUR1523, inserted into the unique *Pst*I site of pGB34 (Fig. 3). Cutting pLC28 with the restriction endonuclease *Cla*I, followed by resection with the exonuclease *Ba*/31 and ligation with T₄ ligase, yielded pLC83 (Fig. 4), which contains the gene for chymosin, but no longer contains a DNA segment with many inconvenient restriction sites. The plasmids pLC28 and pLC83, both in *B. subtilis* 1S-53, were deposited with CBS on July 6, 1983 as Nos. 469.83 and 468.83, respectively.
   In order to place the chymosin gene in phase behind the alpha-amylase gene transcriptional and translational initiation regulatory sequences, pLC83 was digested with *Pst*I*,* resected with nuclease S₁ and ligated with T₄ ligase. The plasmid obtained was called pLC87, and shown to have the correct placement and orientation by sequence analysis. See Figure 5.
c) Construction of the recombinant plasmids pLP33 and pLP87 containing a gene encoding protease (Figures 7 and 8)
   100 µg chromosomal DNA of the protease producing strain *B. subtilis* 168 (Bacillus Genetic Stock Center, Columbus, Ohio) was partially digested with the restriction enzyme *Cla*I. After phenol extraction the DNA fragments were separated on a 1% agarose gel. By means of electroelution several DNA fractions were eluted from the gel and ligated with *Cla*I linearized pGB35 (a recombinant plasmid containing the *Bacillus* vector pGL112, W.M. de Vos. Thesis University of Groningen 1983, and the alpha-amylase gene of pGB33, see Fig. 6). The ligation products were transferred into competent *B. subtilis* RUB331 cells in the manner as described in Example IV. The chloramphenicol-resistant transformamts obtained were analysed for enhanced protease activity (prot⁺) and reduced alpha-amylase production capacity (amy⁻) (see Example IV) on 0.4% casein-amylose plates. Plasmid DNA was isolated from prot⁺amy⁻ transformants and restriction site mapped; pLP33 appears to contain a 3.3 kbp *Cla*I fragment (Fig. 7) which includes a structural gene coding for a serine protease. pLP87 (Fig. 8) has a chromosomal *Cla*l insert of 1.8 kbp containing the genetical information for a non-serine protease.

### Example IV

### Transformation of Bacillus strains

*B. subtilis* 1-85 (trp⁻amy⁻) was transformed by incubating 1 ml cell suspension and 1 µg ligated DNA for 0.5 h at 37°C with gentle shaking (Anagnostopoulos and Spizizen, *J. Bacteriol.* (1961) *81*:741-746). Transformants were selected for antibiotic resistance on minimum medium agar plates, to which 0.02% (w/v) casamino acids (Difco) and 10 µg/ml of an antibiotic were added. These transformants were then analysed for the presence of the desired structural gene.

In the case of alpha-amylase this was performed by looking for halos after covering the plates with a solution containing 0.6% (w/v) KI and 0.3% (w/v) I₂; by positive hydridization to a ³²P labelled DNA probe synthesized *in vitro* in accordance with the N-terminal amino acid sequence of the biologically active enzyme; and by immunoprecipitation with antibodies against the enzyme and by comparative isoelectro-focusing.

In the case of chymosin, positive selection was carried out via hybridization with ³²P labelled pUR1523 DNA and by immuno-precipitation with anti-chymosin antibodies.

To identify the gene for a *Bacillus* protease the transformants were tested for their ability to form halos on casein minimal medium agar plates. The difference between serine and non-serine protease was determined by the method of Scheiner and Quigley *(Anal. Biochemistry* (1982) *122*:58-69)*.*

The selected transformants were used as donor strains in cell fusion experiments.

### Example V

### Cell fusion and regeneration

The transformed *B. subtilis* strain *(B. subtilis* strain 1-85 containing pGB33, *B. subtilis* strain 1S-53 containing pLC87 or *B. subtilis* strain RUB331 containing pLP33) was grown overnight in 50 ml NBSG-X medium (Thorne and Stull, *J. Bacteriol.* (1966) 91:1012-1020 (Table II, page 1014)) with a relevant antibiotic therein at 37°C. The cultures were diluted 1:1 with fresh NBSG-X medium and grown for another 1-1.5 h in the presence of 10 mM iodoacetamide. After centrifuging for 10 min at 5000 rpm in a Sorvall type GSA rotor and resuspending in 10 ml SMM buffer containing 1.5M sucrose, 0.06M MgCl₂ and 0.06M maleate, the cells were protoplasted by incubating the cells for 2 h at 37°C in the presence of 2 mg/ml lysozyme. The protoplasts were spun down (10 min × 5000 rpm), resuspended in 5 ml SMML buffer (L-broth in which 1.5M sucrose, 0.06M MgCl₂ and 0.006M maleate has been dissolved), mixed and repelleted. After being resuspended, the protoplasts were mixed with the protoplasts of the acceptor strain T5, viable cells protoplasted as described above, and incubated for 2 min in the presence of approximately 30% (w/v) of polyethylene glycol 6000. After 1:3 dilution with SMML medium and centrifugation, the pellet was resuspended in a small volume of SMML medium. Then 100 µl aliquots were plated on regeneration agar plates containing 0.7% (w/v) K₂HPO₄, 0.3% (w/v) KH₂PO₄, 0.125% (w/v) (NH₄)₂SO₄, 0.035% (w/v) MgSO₄ · 7H₂O, 1.5% (w/v) agar, 3.7% (w/v) KCI, 0.1% (w/v) glucose, 0.01% (w/v) bovine serum albumin supplemented with 0.1% (w/v) trace element solution containing 0.2% (w/v) MnSO₄, 0.2% (w/v) ZnSO₄, 0.2% (w/v) CoCI₂, 0.5% (w/v) FeSO₄, 6% (w/v) NaCI and 0.5% (w/v) CaCl₂. Moreover these plates contained the relevant antibiotic, in the case of pGB33, pLP33 and pLC87 100-160 µg/ml neomycin. After incubation at 37°C for at least 72h, the plates were replica plated on heart-infusion agar plates, containing also another antibiotic to which the acceptor strain is resistant but to which the donor strain is sensitive. Fusants designated as Type A were analysed according to the methods described in Example IV. In the case of alpha-amylase the procedure was repeated as follows. Type A fusants were fused with *B. subtilis* protoplasts containing pGB36 (a recombinant plasmid of pTL12, containing the gene that encodes resistance for trimethoprim (Tanaka and Kawano, *Gene* (1980) *10*:131-136) and the *Eco*RI restriction fragment of pGB33, containing the gene that codes for *B. licheniformis* alpha-amylase), yielding fusants designated as Type B. Both Type A and Type B fusants were characterized by genomic analysis using ³²P labelled plasmid pGB33 and by comparative SDS gel-electrophoresis. Finally the fusants obtained were selected for the fermentative production of the various enzymes.

### Example VI

### Fermentative production of alpha-amylase, protease and chymosin by genetically engineered Bacillus production strains

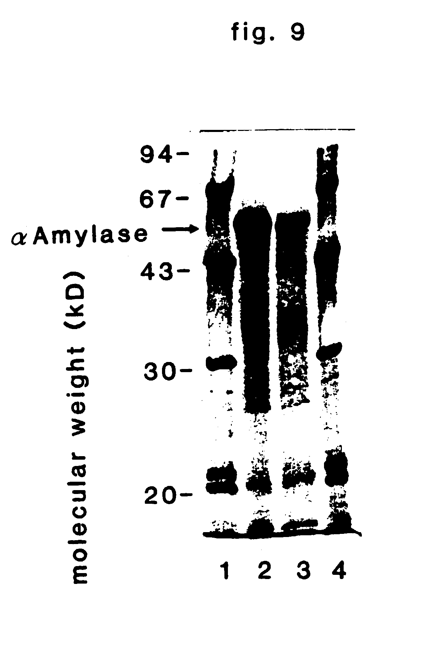

The genetically engineered production strain of *B. licheniformis* T5, obtained by fusion with the *B. subtilis* strain 1-85, containing pGB33 (as described in Example V), was cultivated for 7 days in an industrial nutrient broth under such fermentation conditions that the production of secreted protein is at least 0.5% w/v, alpha-amylase being the major constituent. The production of this engineered strain was compared to the production of the parental strain *B. licheniformis* T5. As evidenced by an SDS polyacrylamide gel-electrophoretogram of products secreted by the production strain *B. licheniformis* T5 (see Fig. 9, lane 3) and those secreted by the genetically engineered strain *B. licheniformis* T5, containing pGB33 (see Fig. 9, lane 2), the production of alpha-amylase was significantly increased by the introduction of the plasmid pGB33.

Comparable results were obtained with the plasmid pLP33, which introduction improved the production of protease and with plasmid pLC87 which introduction resulted in a *B. licheniformis* T5 strain, which is capable of producing chymosin.

Table I shows the summarized results of a quantification of the improvements of the respective genetically engineered microorganisms.

**TABLE 1**

| Organism | plasmid | apha-amylase prod. |
|---|---|---|
| *B. licheniformis* T5 | - | 100% * |
| *B. licheniformis* T5 | pGB 33 | 180% ** |
| *B. lich.* T5 type A(pGB33) | pGB 36 | 230% *** |

| Organism | plasmid | protease production |
|---|---|---|
| *B. licheniformis* T5 | - | 100% |
| *B. licheniformis* T5 | pLP 33 | 145% |

| Organism | plasmid | chymosin production |
|---|---|---|
| *B. licheniformis* T5 | - | - |
| *B. licheniformis* T5 | pLC 87 | + |

| | | |
|---|---|---|
| * 1 alpha-amylase gene; | | |
| ** 2 alpha-amylase genes; | | |
| *** 3 alpha-amylase genes. | | |

The subject method has shown itself as highly successful with *Bacillus.*

It is evident from the above results that a simple and efficient procedure is provided for stably introducing homologous or heterologous genes into industrial *Bacillus* strains, while retaining the desirable characteristics of the strains and providing for the enhanced competence of the cells in the increased production of a desired expression product endogenous to the *Bacillus* host or the production of a novel expression product of interest. High efficiencies of transfer are achieved and where substantial homology exists between the plasmid construct and the industrial *Bacillus* strain chromosome, single or muliple copy integration of the structural genes may be achieved. The subject method greatly enhances the capability of modifying presently existing industrial *Bacillus* strains to provide efficient fermentative production of a wide variety of polypeptides and proteins of interest.

Although the foregoing invention has been described in some detail by way of illustration and example for purposes of clarity of understanding, it will be obvious that certain changes and modifications may be practiced within the scope of the appended claims.

## Claims

1. A method for efficiently introducing into an industrial *Bacillus* strain host DNA capable of stable maintenance and expression of a protein or polypeptide of interest in said industrial host, wherein said industrial *Bacillus* strain host is characterized by being prototrophic, resistant to genetic exchange and phage infection or transformation and secreting DNAses and having a production level of at least 0.5% w/v secreted protein under industrial fermentation conditions, said method comprising:
combining, in the presence of a fusogen, first killed Bacillus host cells which are readily transformable and compatible and contain said DNA as an extrachromosomal element capable of maintenance in said first host and said *Bacillus* industrial strain host cells, under conditions providing for selection of said industrial *Bacillus* strain host cells, wherein said first host cells and said *Bacillus* industrial strain host cells are present as protoplasts, whereby said DNA is transferred to said industrial *Bacillus* strain host cells; and selecting industrial *Bacillus* strain host cells containing said DNA.

2. A method according to Claim 1, wherein said industrial *Bacillus* strain is a *Bacillus licheniformis.*

3. A method according to Claim 1 or 2, wherein said polypeptide is an endogenous enzyme of said *Bacillus* strain.

4. A method according to Claim 3, wherein said endogenous enzyme is an alpha-amylase, preferably a thermostable alpha-amylase, or a protease.

5. A method according to any one of Claims 1-4, wherein the industrial *Bacillus* strain host is resistant to genetic modification by a plasmid, phage or cosmid.

6. A method according to any one of Claims 1-5 for producing an industrial *Bacillus* strain having increased ability to produce a desired protein or polypeptide, said method comprising:
(a) fragmenting DNA containing a gene coding for the desired protein or polypeptide,
(b) ligating a vector to said fragmented DNA,
(c) transforming first host cells with said ligated vector,
(d) selecting the transformed cells for the presence and optionally the expression of the desired gene and optionally inactivating protoplasts of said transformed first host cells,
(e) fusing protoplasts of cells from a selected industrial *Bacillus* host microorganism and said protoplasts of said inactivated transformed first host cells, and
(f) selecting and regenerating the fused cells containing the gene coding for the desired protein or polypeptide and having increased ability to produce the desired protein or polypeptide.

7. The method of Claim 6 wherein the DNA containing a gene coding for the desired protein or polypeptide is fragmented by one or more restriction enzymes.

8. A method for efficiently introducing into an industrial *Bacillus* strain host DNA capable of stable maintenance and expression of a polypeptide of interest in said industrial *Bacillus* strain host, wherein said industrial *Bacillus* strain host is characterized by being prototrophic, resistant to genetic exchange and phage infection or transformation and secreting DNAses and having a production level of at least 0.5% w/v secreted protein under industrial fermentation conditions, said method comprising:
combining, in the presence of a fusogen, first killed *Bacillus* host cells which are readily transformable and compatible and contain said DNA in a plasmid construct having a replication system capable of functioning in *Bacillus* and said industrial *Bacillus* strain host cells, where said cells are present as protoplasts, and whereby said DNA is transferred to said industrial *Bacillus* strain host cells; and selecting industrial *Bacillus* strain host cells containing said DNA.

9. A method according to Claim 8, wherein said industrial *Bacillus* strain host is a *Bacillus licheniformis.*

10. A method according to Claim 8 or 9, wherein said polypeptide of interest is a *Bacillus* endogenous enzyme.

11. A method according to Claim 8, wherein said polypeptide of interest is a *Bacillus* exogenous enzyme.

12. A method according to any one of Claims 8-11, wherein said DNA becomes integrated into the chromosome of said industrial *Bacillus* strain host.

13. A method according to Claim 10, wherein said enzyme is an alpha-amylase preferably a thermostable alpha-amylase, or a protease.

14. A method according to Claim 11, wherein said enzyme is a chymosin.

15. A method for producing a polypeptide of interest in an industrial *Bacillus* strain host, wherein said industrial *Bacillus* strain host is characterized by being prototrophic, resistant to genetic exchange and phage infection or transformation and secreting DNAses and having a production level of at least 0.5% w/v secreted protein under industrial fermentation conditions, said method comprising:
growing industrial *Bacillus* strain host cells prepared according to any one of Claims 1-14 in a suitable nutrient medium; and isolating the polypeptide expressed by said DNA.

16. A method according to Claim 15, wherein said polypeptide is secreted by said industrial *Bacillus* host strain cells and isolated by separation from said nutrient medium.

17. A method according to Claim 16, wherein said secreted polypeptide is an alpha-amylase, preferably a thermostable alpha-amylase, or a protease.

18. The method of Claim 6 wherein the ligated product of step (b) is a plasmid selected from the group consisting of pGB33, deposited with CBS under No. 466.83; pGB34, deposited with CBS under No. 467.83; pLC28, deposited with CBS under No. 469.83; pLC83, deposited with CBS under No. 468.83; and pLC87, obtainable from plasmid pLC83 by *Pst*I digestion. resection with nuclease S₁ and ligation with T₄ ligase.

19. Plasmid pGB33 as contained in *B. subtilis* 1-85, having the deposit accession number CBS 466.83.

20. A genetically engineered industrial microorganism of the genus *Bacillus* which is characterized by being prototrophic, resistant to genetic exchange and phage infection or transformation and secreting DNAses and having a production level of at least 0.5% w/v secreted protein under industrial fermentation conditions, said microorganism comprising inserted DNA in the form of a plasmid construct or a chromosomally integrated plasmid construct or fragments thereof, which DNA is stably maintained in said microorganism said construct containing a gene coding for a desired protein or polypeptide and a selection marker, said genetically engineered microorganism having increased ability to produce said desired protein or polypeptide when compared with the parent industrial microorganism.

21. The genetically engineered industrial *Bacillus* microorganism of Claim 20, which produces alpha-amylase and which contains at least two stable genes coding for alpha-amylase.

22. Use of a genetically engineered industrial *Bacillus* microorganism as defined in Claim 20 or 21 for the production of a protein or polypeptide.

## Patentansprüche

1. Verfahren zur effizienten Einführung in einen industriellen Bacillusstamm-Wirt von DNA, die in dem industriellen Wirt stabil vererbt zu werden vermag und ein interessierendes Protein oder Polypeptid zu exprimieren vermag, wobei der industrielle Bacillusstamm-Wirt dadurch gekennzeichnet ist, dass er unter industriellen Fermentierungsbedingungen prototroph und resistent gegen genetischen Austausch und Phageninfektion oder Transformation ist und DNAsen sekretiert und eine Produktionsleistung von mindestens 0,5 % (Gewicht/Volumen) sekretiertem Protein hat, dadurch gekennzeichnet, dass man in Gegenwart eines Zellfusion vermittelnden Faktors erste, abgetötete Bacillus-Wirtszellen, die leicht transformierbar und kompatibel sind und die die DNA als extrachromosomales Element enthalten, das in dem ersten Wirt vererbt zu werden vermag, und Zellen des industriellen Bacillusstamm-Wirts unter Bedingungen kombiniert, die für die Selektion der Zellen des industriellen Bacillusstamm-Wirts sorgen, wobei die ersten Wirtszellen und die Zellen des industriellen Bacillusstamm-Wirts als Protoplasten vorliegen, wodurch die DNA in die Zellen des industriellen Bacillusstamm-Wirts transferiert wird; und dass man Zellen des industriellen Bacillusstamm-Wirts, die die genannte DNA enthalten, selektioniert.

2. Verfahren nach Anspruch 1, dadurch gekennzeichnet, dass der industrielle Bacillusstamm ein Bacillus licheniformis ist.

3. Verfahren nach Anspruch 1 oder 2, dadurch gekennzeichnet, dass das Polypeptid ein endogenes Enzym des Bacillusstammes ist.

4. Verfahren nach Anspruch 3, dadurch gekennzeichnet, dass das endogene Enzym eine α-Amylase, vorzugsweise eine wärmebeständige α-Amylase, oder eine Protease ist.

5. Verfahren nach einem der Ansprüche 1 bis 4, dadurch gekennzeichnet, dass der industrielle Bacillusstamm-Wirt gegen genetische Modifizierung durch ein Plasmid, einen Phagen oder ein Cosmid resistent ist.

6. Verfahren nach einem der Ansprüche 1 bis 5 zur Erzeugung eines industriellen Bacillusstammes mit erhöhtem Vermögen zum Erzeugen eines gewünschten Proteins oder Polypeptids, dadurch gekennzeichnet, dass man
(a) DNA, die ein Gen enthält, das für das gewünschte Protein oder Polypeptid codiert, fragmentiert,
(b) einen Vektor an die fragmentierte DNA ligiert,
(c) erste Wirtszellen mit dem ligierten Vektor transformiert,
(d) die transformierten Zellen auf das Vorhandensein und gegebenenfalls die Expression des gewünschten Gens selektioniert und gegebenenfalls Protoplasten der transformierten ersten Wirtszellen inaktiviert,
(e) Protoplasten von Zellen aus einem selektionierten industriellen Bacillus-Wirtsmikroorganismus und die genannten Protoplasten der inaktivierten transformierten ersten Wirtszellen verschmilzt und
(f) die verschmolzenen Zellen, die das Gen, das für das gewünschte Protein oder Polypeptid codiert, enthalten und ein erhöhtes Vermögen zum Erzeugen des gewünschten Proteins oder Polypeptids haben, selektioniert und regeneriert.

7. Verfahren nach Anspruch 6, dadurch gekennzeichnet, dass man die DNA, die ein Gen enthält, das für das gewünschte Protein oder Polypeptid codiert, durch eine oder mehrere Restriktionsenzyme fragmentiert.

8. Verfahren zur effizienten Einführung in einen industriellen Bacillusstamm-Wirt von DNA, die in dem industriellen Bacillusstamm-Wirt stabil vererbt zu werden vermag und ein interessierendes Polypeptid zu exprimieren vermag, wobei der industrielle Bacillusstamm-Wirt dadurch gekennzeichnet ist, dass er unter industriellen Fermentierungsbedingungen prototroph und resistent gegen genetischen Austausch und Phageninfektion oder Transformation ist und DNAsen sekretiert und eine Produktionsleistung von mindestens 0,5 % (Gewicht/Volumen) sekretiertem Protein hat, dadurch gekennzeichnet, dass man in Gegenwart eines Zellfusion vermittelnden Faktors erste abgetötete Bacillus-Wirtszellen, die leicht transformierbar und kompatibel sind und die genannte DNA in einem konstruierten Plasmid mit einem Replikationssystem, das in Bacillus zu funktionieren vermag, enthalten, und die genannten Zellen des industriellen Bacillusstamm-Wirts kombiniert, wobei die Zellen als Protoplasten vorliegen, wodurch die genannte DNA in die Zellen des industriellen Bacillusstamm-Wirts transferiert wird; und dass man Zellen des industriellen Bacillusstamm-Wirts, die die genannte DNA enthalten, selektioniert.

9. Verfahren nach Anspruch 8, dadurch gekennzeichnet, dass der industrielle Bacillusstamm-Wirt ein Bacillus licheniformis ist.

10. Verfahren nach Anspruch 8 oder 9, dadurch gekennzeichnet, dass das interessierende Polypeptid ein endogenes Bacillus-Enzym ist.

11. Verfahren nach Anspruch 8, dadurch gekennzeichnet, dass das interessierende Polypeptid ein exogenes Bacillus-Enzym ist.

12. Verfahren nach einem der Ansprüche 8 bis 11, dadurch gekennzeichnet, dass die genannte DNA in das Chromosom des industriellen Bacillusstamm-Wirts integriert wird.

13. Verfahren nach Anspruch 10, dadurch gekennzeichnet, dass das Enzym eine α-Amylase, vorzugsweise eine wärmebeständige α-Amylase, oder eine Protease ist.

14. Verfahren nach Anspruch 11, dadurch gekennzeichnet, dass das Enzym ein Chymosin ist.

15. Verfahren zur Erzeugung eines interessierenden Polypeptides in einem industriellen Bacillusstamm-Wirt, wobei der industrielle Bacillusstamm-Wirt dadurch gekennzeichnet ist, dass er unter industriellen Fermentierungsbedingungen prototroph und resistent gegen genetischen Austausch und Phageninfektion oder Transformation ist und DNAsen sekretiert und eine Produktionsleistung von mindestens 0,5 % (Gewicht/Volumen) sekretiertem Protein hat, dadurch gekennzeichnet, dass man nach einem der Ansprüche 1 bis 14 hergestellte Zellen des industriellen Bacillusstamm-Wirts in einem geeigneten Nährmedium kultiviert; und das durch die genannte DNA exprimierte Polypeptid isoliert.

16. Verfahren nach Anspruch 15, dadurch gekennzeichnet, dass das Polypeptid durch die Zellen des industriellen Bacillusstamm-Wirts sekretiert und durch Abtrennung von dem Nährmedium isoliert wird.

17. Verfahren nach Anspruch 16, dadurch gekennzeichnet, dass das sekretierte Polypeptid eine α(-Amylase, vorzugsweise eine wärmebeständige α(-Amylase, oder eine Protease ist.

18. Verfahren nach Anspruch 6, dadurch gekennzeichnet, dass das ligierte Produkt von Stufe (b) ein Plasmid ist, das aus der Gruppe gewählt ist, die aus pGB33, hinterlegt bei CBS unter der Nummer 466.83; pGB34, hinterlegt bei CBS unter der Nummer 467.83; pLC28, hinterlegt bei CBS unter der Nummer 469.83; pLC83, hinterlegt bei CBS unter der Nummer 468.83; und pLC87, erhältlich aus dem Plasmid pLC83 durch PstI-Verdauung, Abbau der Einzelstrangenden mit Nuclease S₁ und Ligation mit T₄-Ligase, besteht.

19. Plasmid pGB33, wie es in B. subtilis 1-85 mit der Hinterlegungsnummer CBS 466.83 enthalten ist.

20. Genetisch modifizierter industrieller Mikroorganismus der Gattung Baccilus, der dadurch gekennzeichnet ist, dass er unter industriellen Fermentierungsbedingungen prototroph und resistent gegen genetischen Austausch und Phageninfektion oder Transformation ist und DNAsen sekretiert und eine Produktionsleistung von mindestens 0,5 % (Gewicht/Volumen) sekretiertem Protein hat, wobei der Mikroorganismus inserierte DNA in Form eines konstruierten Plasmids oder eines chromosomal integrierten konstruierten Plasmids oder von Fragmenten davon aufweist, wobei die DNA in dem Mikroorganismus stabil gehalten wird und das konstruierte Plasmid ein Gen, das für ein gewünschtes Protein oder Polypeptid codiert, und einen Selektionsmarker enthält, wobei der genetisch modifizierte Mikroorganismus ein erhöhtes Vermögen zur Erzeugung des gewünschten Proteins oder Polypeptids hat, verglichen mit dem industriellen Elternmikroorganismus.

21. Genetisch modifizierter industrieller Bacillus-Mikroorganismus nach Anspruch 20, der α-Amylase erzeugt und der mindestens zwei stabile Gene enthält, die für α-Amylase codieren.

22. Verwendung eines genetisch modifizierten industriellen Bacillus-Mikroorganismus nach Anspruch 20 oder 21 für die Erzeugung eines Proteins oder Polypeptids.

## Revendications

1. Procédé pour introduire efficacement dans une souche hôte de Bacillus industriel de l'ADN capable de maintien stable et d'expression d'une protéine ou d'un polypeptide intéressants dans cet hôte industriel, dans lequel la souche hôte de Bacillus industriel est caractérisée comme étant prototrophe, résistante à l'échange génétique et à l'infection ou à la transformation par des phages et sécrétant des ADNases et ayant un niveau de production d'au moins 0,5% p/v de protéine sécrétée dans des conditions de fermentation industrielles, ce procédé comprenant :
la combinaison, en présence d'un fusogène, de premières cellules hôtes de Bacillus, tuées, qui sont compatibles et aisément transformables et contiennent cet ADN comme élément extrachromosomique capable de maintien dans ce premier hôte et de ces cellules hôtes de la souche de Bacillus industriel, dans des conditions assurant la sélection de ces cellules hôtes de la souche de Bacillus industriel, dans lequel ces premières cellules hôtes et ces cellules hôtes de la souche de Bacillus industriel sont présentes à l'état de protoplastes, de façon que l'ADN soit transféré à ces cellules hôtes de la souche de Bacillus industriel; et la sélection de cellules hôtes de la souche de Bacillus industriel contenant l'ADN.

2. Procédé suivant la revendication 1, dans lequel la souche de Bacillus industriel est un Bacillus licheniformis*.*

3. Procédé suivant la revendication 1 ou 2, dans lequel le polypeptide est une enzyme endogène de la souche de Bacillus.

4. Procédé suivant la revendication 3, dans lequel l'enzyme endogène est une alpha-amylase, de préférence une alpha-amylase thermostable, ou une protéase.

5. Procédé suivant l'une quelconque des revendications 1 à 4, dans lequel la souche hôte de Bacillus industriel est résistante à la modification génétique par un plasmide, phage ou cosmide.

6. Procédé suivant l'une quelconque des revendications 1 à 5, pour produire une souche de Bacillus industriel ayant une aptitude accrue à produire une protéine ou un polypeptide désirés, ce procédé comprenant :
(a) la fragmentation d'un ADN contenant un gène codant pour la protéine ou le polypeptide désirés,
(b) la ligature d'un vecteur à l'ADN fragmenté,
(c) la transformation de premières cellules hôtes par le vecteur ayant subi la ligature,
(d) la sélection des cellules transformées d'après la présence et facultativement, l'expression du gène désiré et facultativement, l'inactivation des protoplastes des premières cellules hôtes transformées,
(e) la fusion de protoplastes de cellules d'un micro-organisme hôte Bacillus industriel sélectionné et des protoplastes des premières cellules hôtes transformées inactivées, et
(f) la sélection et la régénération des cellules fusionnées contenant le gène codant pour la protéine ou le polypeptide désirés et ayant une aptitude accrue à produire la protéine ou le polypeptide désirés.

7. Procédé suivant la revendication 6, dans lequel l'ADN contenant un gène codant pour la protéine ou le polypeptide désirés est fragmenté au moyen d'une ou plusieurs enzymes de restriction.

8. Procédé pour introduire efficacement dans une souche hôte de Bacillus industriel de l'ADN capable de maintien stable et d'expression d'un polypeptide intéressant dans cette souche hôte de Bacillus industriel, dans lequel la souche hôte de Bacillus industriel est caractérisée comme étant prototrophe, résistante à l'échange génétique et à l'infection ou à la transformation par des phages et sécrétant des ADNases et ayant un niveau de production d'au moins 0,5% p/v de protéine sécrétée dans des conditions de fermentation industrielles, ce procédé comprenant :
la combinaison, en présence d'un fusogène, de premières cellules hôtes de Bacillus tuées qui sont aisément transformables et compatibles et qui contiennent cet ADN dans un plasmide construit ayant un système de réplication capable de fonctionner dans Bacillus et les cellules hôtes de la souche de Bacillus industriel, où ces cellules sont présentes à l'état de protoplastes, de façon que l'ADN soit transféré à ces cellules hôtes de la souche de Bacillus industriel; et la sélection de cellules hôtes de la souche de Bacillus industriel contenant cet ADN.

9. Procédé suivant la revendication 8, dans lequel la souche hôte de Bacillus industriel est un Bacillus licheniformis.

10. Procédé suivant la revendication 8 ou 9, dans lequel le polypeptide intéressant est une enzyme endogène de Bacillus.

11. Procédé suivant la revendication 8, dans lequel le polypeptide intéressant est une enzyme exogène de Bacillus.

12. Procédé suivant l'une quelconque des revendications 8 à 11, dans lequel l'ADN s'intègre au chromosome de la souche hôte de Bacillus industriel.

13. Procédé suivant la revendication 10, dans lequel l'enzyme est une alpha-amylase, de préférence une alpha-amylase thermostable, ou une protéase.

14. Procédé suivant la revendication 11, dans lequel l'enzyme est une chymosine.

15. Procédé pour produire un polypeptide intéressant dans une souche hôte de Bacillus industriel, dans lequel cette souche hôte de Bacillus industriel est caractérisée comme étant prototrophe, résistante à l'échange génétique et à l'infection ou à la transformation par des phages et sécrétant des ADNases et ayant un niveau de production d'au moins 0,5% p/v de protéine sécrétée dans des conditions de fermentation industrielles, ce procédé comprenant :
la culture de cellules hôtes de la souche de Bacillus industriel préparées suivant l'une quelconque des revendications 1 à 14 dans un milieu nutritif approprié; et l'isolement du polypeptide exprimé par cet ADN.

16. Procédé suivant la revendication 15, dans lequel le polypeptide est sécrété par les cellules de la souche hôte de Bacillus industriel et isolé par séparation hors du milieu nutritif.

17. Procédé suivant la revendication 16, dans lequel le polypeptide sécrété est une alpha-amylase, de préférence une alpha-amylase thermostable ou une protéase.

18. Procédé suivant la revendication 6, dans lequel le produit de ligature du stade (b) est un plasmide choisi dans la classe formée par pGB33 déposé au CBS sous le n° 466.83; pGB34 déposé au CBS sous le n° 467.83; pLC28 déposé au CBS sous le n° 469.83; pLC83 déposé au CBS sous le n° 468.83; et pLC87 qui peut s'obtenir à partir du plasmide pLC83 par digestion par PstI, coupure par la nucléase S₁ et ligature par la ligase T4.

19. Plasmide pGB33 tel que contenu dans B. subtilis 1-85 ayant le numéro d'accès au dépôt CBS 466.83.

20. Micro-organisme industriel manipulé génétiquement du genre Bacillus qui est caractérisé comme étant prototrophe, résistant à l'échange génétique et à l'infection ou à la transformation par des phages et sécrétant des ADNases et ayant un niveau de production d'au moins 0,5% p/v de protéine sécrétée dans des conditions de fermentation industrielles, ce micro-organisme comprenant de l'ADN inséré sous la forme d'un plasmide construit ou d'un plasmide construit intégré au chromosome ou de fragments de celui-ci, l'ADN étant maintenu de manière stable dans le micro-organisme, ce plasmide construit contenant un gène codant pour une protéine ou un polypeptide désirés et un marqueur de sélection, ce micro-organisme manipulé génétiquement ayant une aptitude accrue à produire la protéine ou le polypeptide désirés, par comparaison avec le micro-organisme industriel parent.

21. Micro-organisme Bacillus industriel manipulé génétiquement suivant la revendication 20, qui produit de l'alpha-amylase et qui contient au moins deux gènes stables codant pour l'alpha-amylase.

22. Utilisation d'un micro-organisme Bacillus industriel manipulé génétiquement tel que défini dans la revendication 20 ou 21, pour la production d'une protéine ou d'un polypeptide.
